# EUROPEAN PATENT SPECIFICATION

(11) **EP 2 083 733 B1**
(45) Date of publication and mention of the grant of the patent: **25.06.2014**
(21) Application number: 07824577.6
(22) Date of filing: 14.11.2007
(51) Int. Cl.: A61B 18/00, H01R 13/52

(54) **ELECTRICAL CONNECTOR FOR ELECTRO-SURGICAL INSTRUMENTS**
ELEKTRISCHES VERBINDUNGSGLIED FÜR ELEKTROCHIRURGISCHE INSTRUMENTE
CONNECTEUR ÉLECTRIQUE POUR INSTRUMENTS ÉLECTRO-CHIRURGICAUX

(30) Priority: 14.11.2006 GB 0622671
(43) Date of publication of application: 05.08.2009
(73) Proprietor: Brooke, Gerard Michael, Stroud Gloucestershire GL5 3TJ (GB)
(72) Inventor: Brooke, Gerard Michael, Stroud Gloucestershire GL5 3TJ (GB)
(74) Representative: Newell, William Joseph
(86) International application number: PCT/GB2007/004354
(87) International publication number: WO 2008/059248

(56) References cited:
- WO-A-00/24329
- WO-A-92/22258
- WO-A-97/24073
- WO-A-02/071965

## Description

This invention relates to electro-surgical instruments.

Such instruments normally comprise a main body portion which is held by the surgeon and which contains electrical drive circuitry, and an electro-surgical implement projecting from the forward end thereof to which the electro-surgical current is applied by the circuit. Such instruments are typically selectively operable to effect cutting or coagulation of tissue by the application of high frequency current via the electro-surgical implement which acts as an electrode. The tissue contacting portion of the implement may take many different forms according to the nature of the surgery involved. Typical forms include a blade, ball and needle in various different shapes and sizes. The implement needs to make good electrical contact with the electrical drive circuitry within the instrument body. The implements need to be readily replaceable or exchangeable and so in a conventional arrangement the rearward portion of the implement is a push-fit into a terminal assembly within the main body. It is of course important to ensure that there is good electrical contact between the implement and the electrical drive circuitry within the body to ensure that the high frequency electric current is passed to the implement. Two different terminations or plugs are available; US implements have a rearward plug which is 2.38mm (³/₁₆ inch) in diameter, whereas the German fitting is a greater diameter of 4mm. Surgeons will often favour particular shapes and sizes of implements of either plug type, and the availability of implements with two different plug types is problematic in terms of inventory control and possible confusion in the operating theatre.

WO00/24329 discloses an electro-surgical device with an elastomeric seal defining a flexible opening that can accommodate varying diameters of electrodes or blades.

Accordingly we have developed an electrical surgical instrument that is capable of taking either type of implement.

In one aspect, this invention provides an electro-surgical instrument comprising a body portion housing an electrical circuit for supplying an electro-surgical current to an output terminal assembly in a forward region thereof, said output terminal assembly being capable of slideably receiving and making electrical contact in use with an electro-surgical implement having a cooperating plug portion of a first shape and/or size, or with an electro-surgical implement having a cooperating plug portion of a second shape and/or size, wherein the output terminal assembly includes an electrically conducting terminal element having an internal bore of given internal diameter adapted slideably to receive a plug portion of an electro-surgical implement of a first type and to make electrical contact therewith, and the output terminal assembly also includes an electrically conducting resilient sleeve in electrical contact with said terminal element and extending forwardly and generally concentrically with respect to said bore, and of larger internal diameter than said bore such that the plug portion of a first type of electro-surgical implement may pass therethrough to slideably engage and make electrical contact with said bore, but the plug portion of said second type of electro-surgical implement is an interference fit with a forward portion of said resilient sleeve to make electrical contact therewith.

In this way, the electro-surgical instrument can be used with either type of electro-surgical implement thereby simplifying operation and inventory control.

In one particular example, the first type of electro-surgical implement has a plug portion of about 2.38mm in outside diameter and the second type of electro-surgical implement has an outer diameter of 4mm.

In this manner good electrical conductivity is assured between the electrical circuit and either type of electro-surgical implement. Furthermore, the instrument can receive either type of electro-surgical implement without any modification, thereby facilitating use of a single type of electro-surgical instrument with either type of implement.

The terminal element may conveniently comprise a metal terminal secured to a printed circuit board defining at least part of said electrical circuit. The electrically conducting resilient sleeve may comprise a rearward part captive to said terminal element and a forward part for receiving plug portions of said second type. The electrically conducting resilient sleeve may be formed out of a resilient sheet metal material.

In existing electro-surgical instruments, the electrical power lead for supplying power to the electrical circuit is permanently attached. This means that both the instrument and the electrical lead have to be discarded at the end of the useful life of the instrument which usually reaches this point well before the end of the operational life of the lead. In a preferred arrangement therefore the instrument includes at a rearward end of said body portion a socket for releasably receiving an electrical power lead.

In one arrangement, the electrical circuit may be provided on a circuit board having input electrical contact regions on one or both surfaces of a rearward end region the board, and the electrical power lead may comprise an edge connector for releasably contacting said electrical contact regions.

It will be appreciated that this design may also be used in electro-surgical instruments which do not have the ability to receive two different types of electro-surgical implement.

The invention also extends to a power lead *per se* for an electro-surgical instrument of the type described above, the power lead comprising a plug for plugging into the socket at the rear end of the body portion and an edge connector for making electrical contact with a circuit disposed within the electro-surgical instrument.

The invention may be performed in various ways, and an example thereof will now be described in detail, reference being made to the accompanying drawings, in which:
Figure 1 is a general perspective view of an electro-surgical instrument in accordance with this invention;
Figure 2 is an exploded perspective view of the printed circuit board, the connector, and an electro-surgical implement having a first type of plug, and an electro-surgical implement having a second type of plug;
Figure 3 is a side view of the arrangement shown in Figure 2;
Figure 4 is a cross-sectional view of the arrangement shown in Figure 2;
Figure 5(a) is an enlarged sectional view showing an implement with a first type of plug connected to the printed circuit board, and Figure 5(b) is a similar view showing an implement with the second type of plug connected to the printed circuit board;
Figure 6 is an enlarged perspective view of a resilient sleeve connector;
Figure 7 is an enlarged section view of the resilient sleeve connector;
Figure 8 is a schematic view showing the electrical lead disconnected from the printed circuit board in the electro-surgical instrument, and
Figures 9(a) and (b) are respective perspective, side and end views of the edge connector at the end of the electrical lead.

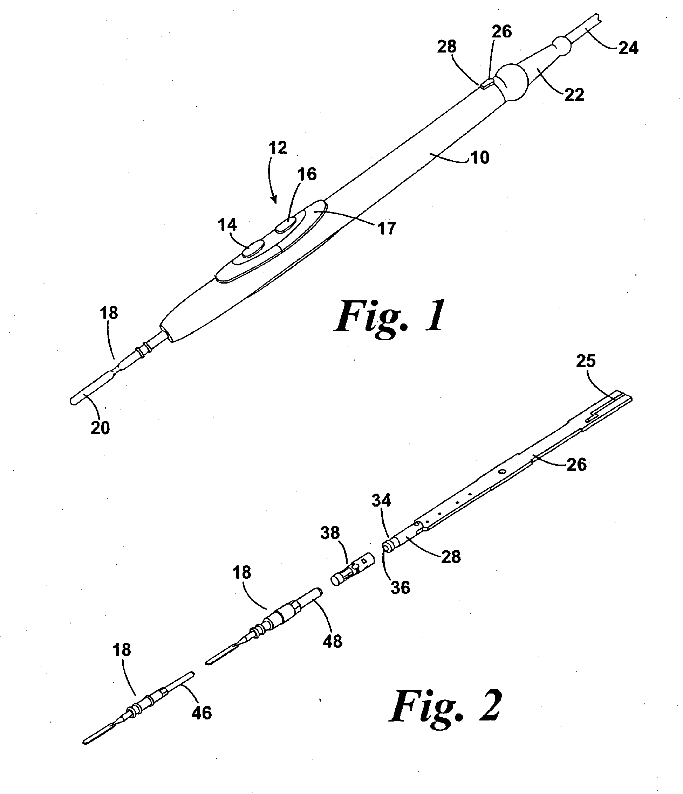

Referring to Figure 1, the electro-surgical instrument of this invention comprises a pen-like body portion 10 having a switch assembly 12 including a cut switch 14 and a cauterise switch 16. The switches 14 and 16 are integrally sealed to the body portion 10 by an over-moulded flange 17. At its forward end the body portion has an aperture in which is fitted an electro-surgical implement 18 which has a forward exposed electrically conducting portion 20 and at its other end (not seen) makes electrical contact with a drive circuit (not seen) as to be described below. At its rear end, the main body portion has a socket which releasably receives the plug 22 of a power lead 24, as to be described below.

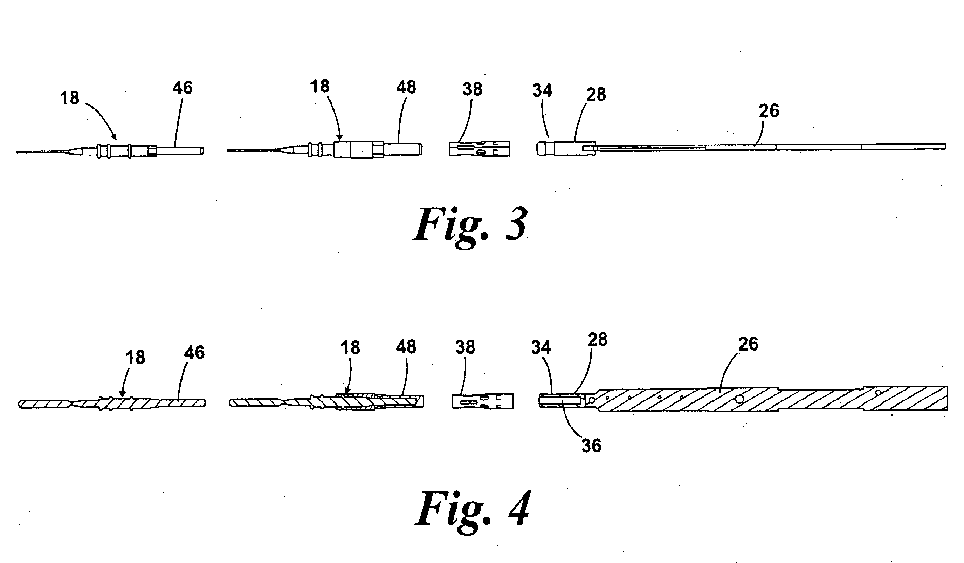

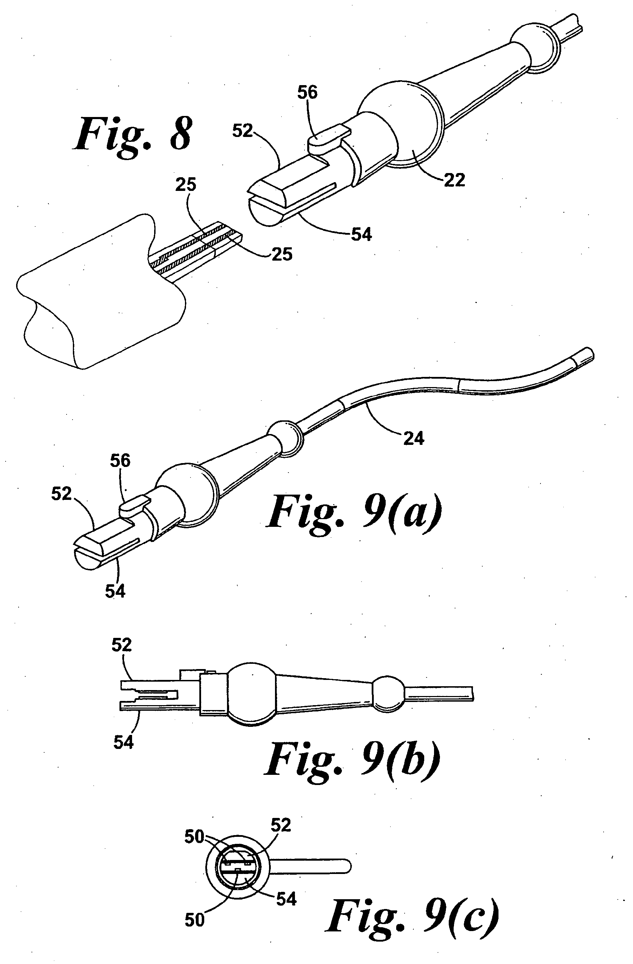

Referring now to Figures 2 to 7, the main body houses a drive circuit disposed on a printed circuit board 26. At its forward end the printed circuit board has a tubular electrical contact 28 which receives the electro-surgical current. At its rear end, the printed circuit board has electrical contact regions 25 or tracks for receiving power from the power lead 24. In this example there is one track 25 on one side of the printed circuit board and two on the other (see Figure 8).

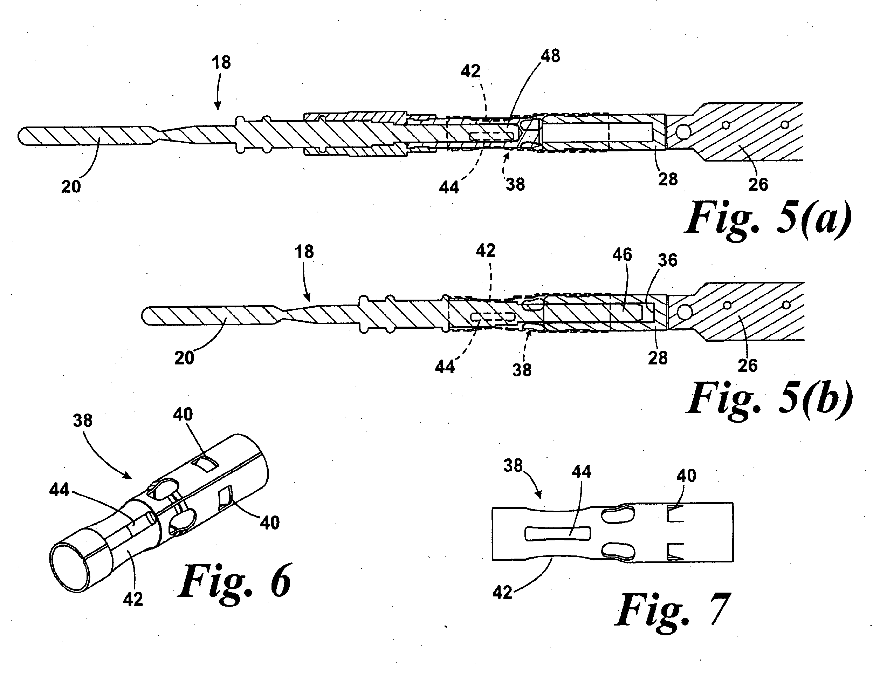

The tubular terminal 28 has an external circumferential groove 34 a little back from its front end and an internal bore 36. A sprung metal sleeve connector 38 has spring tangs 40 (see Figure 7), which allow the sleeve 38 to be securely clipped in good electrical contact with the tubular terminal 28 (as seen in Figures 5(a) and (b)). Forwardly of the tangs 40 the sleeve has a waisted portion 42 which is made springy by slots 44 before terminating in a forward collar region of substantially the same diameter as the rearward region.

As is seen in Figures 2 to 5, electro-surgical implements 18 are available with two different types of electrically conducting plugs 46 and 48. In the first type the external diameter of the plug 48 is 2.38mm and in the second type the plug 46 is 4mm. Referring now especially to Figures 5(a) and (b), the device illustrated in the Figures is adapted to allow either type of implement to be fitted into the instrument. This is achieved by careful selection of the diameter of the waisted portion 42 of the sleeve 38 so that, when an implement of the second type with the larger of plug 48 is push-fitted into the forward end of the instrument, the plug 48 is an interference fit with the waisted portion of the resilient sleeve. However, when an electro-surgical implement of the first type having the smaller plug 46 is push-fitted into the instrument, the plug portion 46 passes through and beyond the resilient sleeve to be slideably received in good electrical contact with the bore 36 in the tubular element 28, as seen in Figure 5(b).

Referring now to Figures 8 and 9, as previously mentioned the printed circuit board has three contacts 25, two on one surface and one on the other. The plug 22 on the power lead 24 comprises a bifurcated portion which correspondingly has two contacts 50 on one limb 52 and one on the other 54. The plug 22 is push-fitted into the rear end of the body portion 10 with the correct orientation being determined by a rib 56 on the plug 22 and a corresponding notch 58 on the body portion 10. Pushing the plug home ensures that the contacts on the plug make good electrical contact with the respective contacts on the printed circuit board.

## Claims

1. An electro-surgical instrument comprising a body portion (10) housing an electrical circuit for supplying an electro-surgical current to an output terminal assembly (28) in a forward region thereof, said output terminal assembly being capable of slideably receiving and making electrical contact in use with an electro-surgical implement (18) having a cooperating plug portion (46) of a first shape and/or size, or with an electro-surgical implement having a cooperating plug portion of a second shape and/or size, wherein the output terminal assembly (28) includes an electrically conducting terminal element having an internal bore (36) of given internal diameter adapted slideably to receive a plug portion (46) of an electro-surgical implement of a first type and to make electrical contact therewith, **characterised in that** the output terminal assembly (28) also includes an electrically conducting resilient sleeve (38) in electrical contact with said terminal element and extending forwardly and generally concentrically with respect to said bore (36), and of larger internal diameter than said bore (36) such that the plug portion (46) of a first type of electro-surgical implement may pass therethrough to slideably engage and make electrical contact with said bore (36), but the plug portion (48) of said second type of electro-surgical implement is an interference fit with a forward portion of said resilient sleeve to make electrical contact therewith (38).

2. An electro-surgical instrument according to Claim 1, wherein the first type of electro-surgical implement has a plug portion of about 2.38mm in outside diameter, and the second type of electro-surgical implement has a plug portion of about 4mm in outside diameter.

3. An electro-surgical instrument according to Claim 1 or Claim 2, wherein said terminal element (28) comprises a metal terminal secured to a printed circuit board (26) defining at least part of said electrical circuit.

4. An electro-surgical instrument according to any of the preceding Claims, wherein the electrically conducting resilient sleeve (38) comprises a rearward part captive to said terminal element and a forward part for receiving plug portions of said second type.

5. An electro-surgical instrument according to any of the preceding Claims, wherein the electrically conducting resilient sleeve (38) is formed out of a resilient sheet metal material.

6. An electro-surgical instrument according to any of the preceding Claims, wherein the instrument includes at a rearward end of said body portion a socket for releasably receiving an electrical power lead (22, 24).

7. An electro-surgical instrument according to Claim 6, wherein said electrical circuit is provided on a circuit board (26) having input electrical contact regions (25) on one or both of the upper and lower surfaces of a rearward end region of the board, and the electrical power lead comprises an edge connector (52, 54) for releasably contacting said electrical contact regions.

8. An electro-surgical instrument according to Claim 3, wherein the printed circuit board is provided with electrical contact regions on one or both of the upper and lower surfaces of the reward end and regions thereof.

9. An electro-surgical power lead for use with an electro-surgical instrument as claimed in any of the preceding Claims, the power lead comprising a plug (22) for plugging into the socket at the rear end of the body portion of the instrument and an edge connector (52, 54) for making electrical contact with a circuit disposed within the electro-surgical instrument.

## Patentansprüche

1. Elektrochirurgisches Instrument, umfassend einen Körperteil (10), der eine elektrische Schaltung beinhaltet, die dazu dient, einen elektrochirurgischen Strom an eine Ausgangsklemmen-Anordnung (28) in ihrem vorderen Bereich zu liefern, wobei die Ausgangsklemmen-Anordnung einen elektrochirurgischen Körper (18) mit einem zusammenwirkenden Steckerteil (46) einer ersten Form und/oder Größe verschiebbar aufnehmen kann und bei Gebrauch elektrischen Kontakt damit herstellen kann, oder mit einem elektrochirurgischen Körper (18) mit einem zusammenwirkenden Steckerteil (46) einer zweiten Form und/oder Größe, wobei die Ausgangsklemmen-Anordnung (28) mit einem elektrisch leitenden Ausgangsklemmen-Element versehen ist, das eine innere Bohrung (36) gegebenen inneren Durchmessers aufweist, die in der Lage ist, einen Steckerteil (46) eines elektrochirurgischen Körpers eines ersten Typs verschiebbar aufzunehmen und mit ihm einen elektrischen Kontakt herzustellen, **dadurch gekennzeichnet, daß** die Ausgangsklemmen-Anordnung (28) auch eine elektrisch leitende elastische Hülse (38) aufweist, die mit dem Anschlußklemmen-Element in elektrischem Kontakt steht und sich nach vorne erstreckt und im allgemeinen konzentrisch bezüglich der Bohrung (36) angeordnet ist sowie einen größeren Innendurchmesser hat als die Bohrung (36), so daß der Steckerteil (46) einer ersten Art des elektrochirurgischen Körpers hindurch passen kann, um die genannte Bohrung (36) gleitend zu berühren und mit ihr in elektrischen Kontakt zu treten, während der Steckerteil (48) des zweiten Typs des elektrochirurgischen Körpers mit einem vorderen Teil der elastischen Hülse zur Herstellung eines elektrischen Kontaktes mit dieser Hülse (38) einen festen Sitz hat.

2. Elektrochirurgisches Instrument nach Anspruch 1, wobei der erste Typ des elektrochirurgischen Körpers einen Steckerteil mit einem Außendurchmesser von etwa 2,38 mm hat, und der zweite Typ des elektrochirurgischen Körpers einen Steckerteil mit einem Außendurchmesser von etwa 4 mm hat.

3. Elektrochirurgisches Instrument nach Anspruch 1 oder 2, wobei das Ausgangsklemmen-Element (28) eine metallische Anschlußklemme aufweist, die an einer gedruckten Schaltungstafel (26) befestigt ist, welche wenigstens einen Teil der elektrischen Schaltung bildet.

4. Elektrochirurgisches Instrument nach einem der vorhergehenden Ansprüche, wobei die elektrisch leitende, elastische Hülse (38) einen rückwärtigen Teil aufweist, der an dem Ausgangsklemmen-Element gesichert ist, sowie einen vorderen Teil zur Aufnahme von Steckerteilen des zweiten Typs.

5. Elektrochirurgisches Instrument nach einem der vorhergehenden Ansprüche, wobei die elektrisch leitende, elastische Hülse (38) aus einem federnden Metallblech-Material geformt ist.

6. Elektrochirurgisches Instrument nach einem der vorhergehenden Ansprüche, wobei das Instrument an einem rückwärtigen Ende des Körperteils eine Fassung zur lösbaren Aufnahme einer elektrischen Stromleitung (22, 24) aufweist.

7. Elektrochirurgisches Instrument nach Anspruch 6, wobei die elektrische Schaltung auf einer Schaltungstafel (26) ausgebildet ist, welche elektrische Eingangskontakt-Bereiche (25) auf einer oder beiden oberen und unteren Oberflächen eines rückwärtigen Endbereiches der Tafel besitzt, und wobei die elektrische Stromleitung einen Steckverbinder (52, 54) zur lösbaren Kontaktierung der elektrischen Kontaktbereiche aufweist.

8. Elektrochirurgisches Instrument nach Anspruch 3, wobei die gedruckte Schaltungstafel mit elektrischen Kontaktbereichen auf der einen oder auf beiden der oberen und unteren Oberflächen des rückwärtigen Endes und ihrer Zonen versehen ist.

9. Elektrochirurgische Stromleitung zur Verwendung mit einem elektrochirurgischen Instrument nach einem der vorhergehenden Ansprüche, wobei die Stromleitung einen Stecker (22) zum Einstecken in die Fassung an dem hinteren Ende des Körperteils des Instruments aufweist, sowie einen Steckverbinder (52, 54), durch den mit einer Schaltung elektrischer Kontakt hergestellt werden kann, die sich in dem elektrochirurgischen Instrument befindet.

## Revendications

1. Instrument électro-chirurgical comprenant une partie de corps (10) recevant un circuit électrique pour fournir un courant électro-chirurgical à un ensemble borne de sortie (28) dans une région avant de celui-ci, ledit ensemble borne de sortie étant apte à recevoir de manière coulissante et à réaliser un contact électrique en utilisation avec un équipement électro-chirurgical (18) ayant une partie de fiche de coopération (46) d'une première forme et/ou taille, ou avec un équipement électro-chirurgical ayant une partie de fiche de coopération d'une seconde forme et/ou taille, dans lequel l'ensemble borne de sortie (28) comprend un élément de borne conducteur de l'électricité ayant un alésage interne (36) de diamètre interne donné adapté pour recevoir de manière coulissante une partie de fiche (46) d'un équipement électro-chirurgical d'un premier type et pour réaliser un contact électrique avec celle-ci, **caractérisé par le fait que** l'ensemble borne de sortie (28) comprend également un manchon résilient conducteur de l'électricité (38) en contact électrique avec ledit élément de borne et s'étendant vers l'avant et généralement de manière concentrique par rapport audit alésage (36), et de diamètre interne plus grand que ledit alésage (36), de telle sorte que la partie de fiche (46) d'un premier type d'équipement électro-chirurgical peut passer à travers celui-ci pour s'engager de manière coulissante et réaliser un contact électrique avec ledit alésage (36), mais que la partie de fiche (48) dudit second type d'équipement électro-chirurgical est à ajustement serré avec une partie avant dudit manchon résilient pour réaliser un contact électrique avec celui-ci (38).

2. Instrument électro-chirurgical selon la revendication 1, dans lequel le premier type d'équipement électro-chirurgical possède une partie de fiche d'environ 2,38 mm de diamètre extérieur, et le second type d'équipement électro-chirurgical possède une partie de fiche d'environ 4 mm de diamètre extérieur.

3. Instrument électro-chirurgical selon la revendication 1 ou la revendication 2, dans lequel ledit élément de borne (28) comprend une borne métallique fixée sur une carte à circuits imprimés (26) définissant au moins une partie dudit circuit électrique.

4. Instrument électro-chirurgical selon l'une quelconque des revendications précédentes, dans lequel le manchon résilient conducteur de l'électricité (38) comprend une partie arrière captive dudit élément de borne et une partie avant pour recevoir des parties de fiche dudit second type.

5. Instrument électro-chirurgical selon l'une quelconque des revendications précédentes, dans lequel le manchon résilient conducteur de l'électricité (38) est formé à partir d'un matériau métallique en feuille résilient.

6. Instrument électro-chirurgical selon l'une quelconque des revendications précédentes, dans lequel l'instrument comprend au niveau d'une extrémité arrière de ladite partie de corps une prise pour recevoir de manière libérable un fil conducteur d'alimentation électrique (22, 24).

7. Instrument électro-chirurgical selon la revendication 6, dans lequel ledit circuit électrique est disposé sur une carte à circuits (26) ayant des régions de contact électrique d'entrée (25) sur l'une ou les deux des surfaces supérieure et inférieure d'une région d'extrémité arrière de la carte, et le fil conducteur d'alimentation électrique comprend un connecteur de bord (52, 54) pour mettre en contact de manière libérable lesdites régions de contact électrique.

8. Instrument électro-chirurgical selon la revendication 3, dans lequel la carte à circuits imprimés comporte des régions de contact électrique sur l'une ou les deux des surfaces supérieure et inférieure des régions d'extrémité arrière de celle-ci.

9. Fil conducteur d'alimentation électro-chirurgical pour une utilisation avec un instrument électro-chirurgical tel que revendiqué à l'une quelconque des revendications précédentes, le fil conducteur d'alimentation comprenant une fiche (22) pour un branchement dans la prise au niveau de l'extrémité arrière de la partie de corps de l'instrument et un connecteur de bord (52, 54) pour réaliser un contact électrique avec un circuit disposé à l'intérieur de l'instrument électro-chirurgical.
